# EUROPEAN PATENT SPECIFICATION

(11) **EP 3 294 271 B1**
(45) Date of publication and mention of the grant of the patent: **01.07.2020**
(21) Application number: 16725783.1
(22) Date of filing: 11.05.2016
(51) Int. Cl.: A61K 9/48, A61Q 11/00, A61K 36/45, A61K 31/315, A61K 31/733, A23P 10/30, A23L 33/105, A61K 8/27, A61K 36/82, A61K 8/65, A61K 8/73, A61K 8/92, A61K 8/11, A61K 31/718, A61K 31/734, A61K 33/30, A61K 45/06

(54) **SOFT CAPSULE AND USE IN THE TREATMENT OF HALITOSIS**
WEICHKAPSEL UND VERWENDUNG BEI DER BEHANDLUNG VON MUNDGERUCH
CAPSULE MOLLE ET UTILISATION DANS LE TRAITEMENT DE LA MAUVAISE HALEINE

(30) Priority: 11.05.2015 BE 201505294
(43) Date of publication of application: 21.03.2018
(73) Proprietor: Sylphar NV, 9831 Deurle (BE)
(72) Inventor: LIST, Robin, 9850 Hansbeke (BE)
(74) Representative: De Clercq & Partners
(86) International application number: PCT/EP2016/060492
(87) International publication number: WO 2016/180848

(56) References cited:
- EP-A1- 1 020 177
- WO-A1-01/19204
- Dr Vidya Dodwad ET AL: "P -46 International Journal of Pharma and Bio Sciences HERBAL MOUTHWASHES - A GIFT OF NATURE 1 DR. BHAVNA JHA KUKREJA AND 2 NATURAL CHEMISTRY RESEARCH ARTICLE", , 1 June 2012 (2012-06-01), XP055201068, Retrieved from the Internet: URL:http://ijpbs.net/vol-3/issue-2/pharma/ 6.pdf [retrieved on 2015-07-08]

## Description

### FIELD OF THE INVENTION

The invention is broadly in the medical field, more precisely in the field of dietary supplements useful for the treatment of halitosis. In particular, the invention concerns a soft capsule and its use in the treatment of halitosis.

### BACKGROUND OF THE INVENTION

Breath malodour, also referred to as halitosis, is a significant social and psychological handicap. Up to 50% of the population can suffer from oral malodour. The causes and intensity of the oral malodour may vary. The primary origin of halitosis is the result of microbial putrefaction accompanied by the release of volatile sulphur compounds (VSCs), for instance hydrogen sulphide (H₂S), methyl mercaptan (CH₃SH), and dimethyl sulphide (CH₃)₂S, and other volatile compounds such as butyric or propionic acid, and diamines.

The causes of halitosis may be exogenous and may relate to lack of saliva flow while sleeping, consumption of certain foods such as garlic and onion, consumption of certain drinks such as alcohol and dairy products, and/or smoking. The causes of halitosis may also be endogenous in which case the halitosis is also referred to as true oral halitosis. True oral halitosis can be related to disorders of the oral cavity such as poor oral hygiene or gingivitis; disorders of the respiratory tract; disorders of gastrointestinal (GI) tract, or to drugs. Halitosis may also have psychogenic causes.

Commercial mouthrinses comprise antimicrobial agents such as chlorhexidine and cetylpyridinium chloride (CPC). However, such mouthrinses have major drawbacks, namely they cause dental discoloration, alterations in taste, and increased calculus formation, they have only short term usability, and they kill all oral bacteria, sometimes aggravating the problem. These products also do not provide a long-term treatment of the breath malodour.

EP1020177 provides a multi-layered soft capsule for eliminating bad breath comprising a first soft capsule composed of a first soft capsule layer and a first soft capsule content, and a second soft capsule composed of a second soft capsule layer and a second soft capsule content, which is contained in the first soft capsule. An exemplary multi-layered soft capsule of EP1020177 contains a first soft capsule containing gelatin, aspartame, sorbitol, lemon oil, peppermint, L-menthol, winter-green oil, vanilla oil, and palm oil, and a second soft capsule containing gelatin, aspartame, parsley oil, and palm oil.

In view of the above, there remains a need in the art for further and/or improved products for use in the treatment of halitosis.

### SUMMARY OF THE INVENTION

The present inventors have found pharmaceutical formulations, in particular soft capsules, addressing one or more of the above-mentioned problems in the art.

The present invention provides subject-matter as set forth in any one and all of the appended claims 1 to 16.

Accordingly, a first aspect of the invention relates to a soft capsule (i.e., capsule-in-capsule) comprising (a) a first soft capsule configured for targeted delivery to the mouth and (b) a second soft capsule configured for targeted delivery to the stomach, wherein the first soft capsule comprises one or more active ingredients capable of blocking adhesion of harmful bacteria to the oral cavity, a metal salt, and a prebiotic fibre, wherein the one or more active ingredients capable of blocking adhesion of harmful bacteria to the oral cavity are selected from the group consisting of cranberry extract, wild blueberry extract, strawberry extract, tea tree oil, and chamomile oil, and wherein the metal salt is a zinc salt; the second soft capsule comprises one or more active ingredients capable of blocking adhesion of harmful bacteria to the stomach and an alginate, wherein the one or more active ingredients capable of blocking adhesion of harmful bacteria to the stomach are selected from the group consisting of cranberry extract, wild blueberry extract, strawberry extract, tea tree oil, chamomile oil, tea extract, neem stick extract, snowdrop extract, seaweed extract, hop bract extract, Galla chinensis extract, avocado, and legume storage proteins; and the second soft capsule is contained in the first soft capsule.

Such soft capsules advantageously allow a targeted and sustained therapeutic delivery of the active ingredients in the mouth followed by a targeted and therapeutic delivery of the active ingredients in the stomach, thereby allowing an effective instant and long-term treatment of halitosis. The soft capsules illustrating the principles of the present invention allow an instant effect and a long-term effect by treatment of halitosis in the mouth and in the gastrointestinal tract, in particular in the stomach. These effects are obtained without the side effects of currently available OTC drugs. Advantageously, the active ingredients in the first soft capsule effectively allow neutralization of undesired bacteria and provide prebiotic stimulation of good oral microflora, without any side effects such as dental discoloration. The active ingredients in the second soft capsule also effectively allow neutralization of undesired bacteria and form a protective layer on top of the stomach content, thereby inhibiting gastric reflux.

A second aspect relates to the soft capsules as taught herein, for use as a medicament.

A further aspect relates to the soft capsules as taught herein, for use in the treatment of halitosis, preferably wherein the treatment comprises an oral treatment of halitosis and a gastrointestinal treatment of halitosis.

A further aspect relates to a method for preparing the soft capsules as taught herein, comprising simultaneously extruding a composite jet stream into a cooling solution, wherein the composite jet stream consists of a second soft capsule filling solution extruded through a first nozzle, a second soft capsule layer-forming solution though a second nozzle, a first soft capsule filling solution extruded through a third nozzle and a first soft capsule layer-forming solution extruded through a fourth nozzle, with the first, second, third and fourth nozzles being arranged concentrically, a diameter of the nozzles gradually increasing in a numerical order, and wherein the first soft capsule layer-forming solution comprises a metal salt, wherein the metal salt is a zinc salt; the first soft capsule filling solution comprises one or more active ingredients capable of blocking adhesion of harmful bacteria to the oral cavity and a prebiotic fibre, wherein the one or more active ingredients capable of blocking adhesion of harmful bacteria to the oral cavity are selected from the group consisting of cranberry extract, wild blueberry extract, strawberry extract, tea tree oil, and chamomile oil; the second soft capsule layer-forming solution comprises an alginate; and the second soft capsule filling solution comprises one or more active ingredients capable of blocking adhesion of harmful bacteria to the stomach, wherein the one or more active ingredients capable of blocking adhesion of harmful bacteria to the stomach are selected from the group consisting of cranberry extract, wild blueberry extract, strawberry extract, tea tree oil, chamomile oil, tea extract, neem stick extract, snowdrop extract, seaweed extract, hop bract extract, Galla chinensis extract, avocado, and legume storage proteins.

The above and further aspects and preferred embodiments of the invention are described in the following sections and in the appended claims. The subject-matter of appended claims is hereby specifically incorporated in this specification.

### DETAILED DESCRIPTION OF THE INVENTION

As used herein, the singular forms "a", "an", and "the" include both singular and plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps. The terms also encompass "consisting of' and "consisting essentially of'.

The recitation of numerical ranges by endpoints includes all numbers and fractions subsumed within the respective ranges, as well as the recited endpoints.

The term "about" as used herein when referring to a measurable value such as a parameter, an amount, a temporal duration, and the like, is meant to encompass variations of and from the specified value, in particular variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

Whereas the term "one or more", such as one or more members of a group of members, is clear per se, by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

All documents cited in the present specification are hereby incorporated by reference in their entirety.

Unless otherwise specified, all terms used in disclosing the invention, including technical and scientific terms, have the meaning as commonly understood by one of ordinary skill in the art to which this invention belongs. By means of further guidance, term definitions may be included to better appreciate the teaching of the present invention.

The present inventors have found a therapeutic composition comprising a unique combination of active ingredients to effectively treat and prevent halitosis. In the context of the present invention, the therapeutic composition is formulated in a soft capsule.

Accordingly, a first aspect of the invention relates to a soft capsule comprising (a) a first soft capsule configured for targeted delivery to the mouth and (b) a second soft capsule configured for targeted delivery to the stomach, wherein the first soft capsule comprises one or more active ingredients capable of blocking adhesion of harmful bacteria to the oral cavity, a metal salt, and a prebiotic fibre, wherein the one or more active ingredients capable of blocking adhesion of harmful bacteria to the oral cavity are selected from the group consisting of cranberry extract, wild blueberry extract, strawberry extract, tea tree oil, and chamomile oil, and wherein the metal salt is a zinc salt; the second soft capsule comprises one or more active ingredients capable of blocking adhesion of harmful bacteria to the stomach and an alginate, wherein the one or more active ingredients capable of blocking adhesion of harmful bacteria to the stomach are selected from the group consisting of cranberry extract, wild blueberry extract, strawberry extract, tea tree oil, chamomile oil, tea extract, neem stick extract, snowdrop extract, seaweed extract, hop bract extract, Galla chinensis extract, avocado, and legume storage proteins; and the second soft capsule is contained in the first soft capsule.

The terms "pharmaceutical formulation", "pharmaceutical composition", or "pharmaceutical preparation" may be used interchangeably herein. Likewise, the terms "formulation", "composition", or "preparation" may be used interchangeably herein.

In the context of the present invention, the soft capsule as taught herein comprises a first soft capsule configured for targeted delivery to the mouth (i.e., outer capsule) and a second soft capsule configured for targeted delivery to the stomach (i.e., inner capsule), wherein the second soft capsule is contained in the first soft capsule.

The first soft capsule as taught herein is configured for targeted delivery to the mouth.

The recitation "configured for targeted delivery to the mouth" as used herein refers to the capability of the first soft capsule to be soluble in the oral cavity. The solubility of the first soft capsule advantageously allows releasing the active ingredient capable of blocking adhesion of harmful bacteria to the oral cavity, the zinc salt, and the prebiotic fibre in the oral cavity.

In certain embodiments, the first soft capsule as taught herein may be dissolved in the oral cavity by masticating the first soft capsule and/or by contacting or mixing the first soft capsule with saliva. In certain embodiments, the first soft capsule as taught herein opens in the oral cavity, thereby advantageously releasing the active ingredients having an effect in the oral cavity.

The second soft capsule as taught herein is configured for targeted delivery to the stomach.

The recitation "configured for targeted delivery to the stomach" as used herein refers to the capability of the second soft capsule to be soluble in a stomach. The solubility of the second soft capsule advantageously allows releasing the active ingredient capable of blocking adhesion of harmful bacteria to the stomach and an alginate in the stomach.

In certain embodiments, the second soft capsule as taught herein may be dissolved in the gastrointestinal tract, in particular in the stomach, by contacting the second soft capsule with gastric acid. In certain embodiments, the second soft capsule as taught herein opens in the stomach, thereby advantageously releasing the active ingredients having an effect in the stomach.

In certain embodiments, the second soft capsule as taught herein may not be soluble in the oral cavity. In certain embodiments, the second soft capsule as taught herein may not be dissolved in the oral cavity by masticating the second soft capsule and/or by contacting or mixing the second soft capsule with saliva. Usually, once the first soft capsule has dissolved in the oral cavity, the remaining second soft capsule will be swallowed by the subject (due to the size of the second soft capsule).

The terms "oral cavity" or "mouth" can be used interchangeably and generally refer to the first portion of the alimentary canal that receives food and saliva. The inside of the mouth is lined with a mucous membrane, the oral mucosa.

The term "stomach" generally refers to the part of the digestive system located between the oesophagus and the small intestine which functions as an important organ of the digestive tract. It is involved in the second phase of digestion, following mastication (i.e., chewing). Like the other parts of the gastrointestinal tract, the stomach wall is lined with mucosa.

The present soft capsule comprising (a) a first soft capsule configured for targeted delivery to the mouth and (b) a second soft capsule configured for targeted delivery to the stomach has two effects of eliminating bad breath or treating halitosis, including an immediate effect and a long-lasting effect, by administration, preferably oral administration, of one capsule. The active ingredient capable of blocking adhesion of harmful bacteria to the oral cavity, the zinc salt, and the prebiotic fibre, which are contained in the first soft capsule, can immediately eliminate the odour after oral administration and allow stimulating the growth and/or activity of beneficial bacteria, thereby contributing to the long-lasting effect of treating halitosis. The first soft capsule may also contain a flavouring component so that a bitter flavour is sweetened when the capsule is dissolved and masticated in the oral cavity, and therefore, the soft capsule has excellent palatability. The second soft capsule comprising the active ingredient capable of blocking adhesion of harmful bacteria to the stomach and the alginate, reaches the stomach without dissolving and masticating in the oral cavity, as well as without mixing with saliva or other peptic juice, and then dissolves in a stomach to show an effect of eliminating "returned odour" and to neutralize harmful bacteria in the stomach.

The active ingredients as taught herein are formulated in a soft capsule.

The terms "soft capsule" or "soft-shelled capsule" can be used interchangeably and typically refer to a solid dosage form in which the active ingredient(s) is (are) enclosed in a soft container or shell. The soft container or shell is typically made of a material capable of forming a soft capsule such as gelatine, agar, methylcellulose, hypromellose or hydroxypropylmethylcellulose (HPMC), or a mixture of any two or more thereof.

The terms "active ingredient" or "active component" can be used interchangeably and broadly refer to a compound or substance which, when provided in an effective amount, achieves a desired therapeutic and/or prophylactic outcome(s). Typically, an active ingredient may achieve such outcome(s) through interacting with and/or modulating living cells or organisms.

The term "active" in the recitations "active ingredient" or "active component" refers to "pharmacologically active" and/or "physically active".

In the present soft capsules, the first soft capsule as taught herein comprises an active ingredient capable of blocking adhesion of harmful bacteria to the oral cavity (e.g., to oral tissue such as the oral mucosa), a metal salt, and a prebiotic fibre, wherein the active ingredient capable of blocking adhesion of harmful bacteria to the oral cavity is selected from the group consisting of cranberry extract, wild blueberry extract, strawberry extract, tea tree oil, and chamomile oil, and wherein the metal salt is a zinc salt.

The active ingredient capable of blocking adhesion of harmful bacteria to the oral cavity in the first soft capsule as taught herein advantageously neutralizes harmful bacteria in the mouth. The active ingredient capable of blocking adhesion of harmful bacteria to the oral cavity adheres to the harmful bacteria without destroying them, thereby blocking bacterial adhesion to the oral mucosa while advantageously preserving immunogenicity. The natural immune system of the subject can react to the harmful bacteria, thereby allowing that the subject develops immunity.

The zinc salt in the first soft capsule as taught herein advantageously allows eliminating volatile malodour gases by capturing Volatile Sulphur Compounds (VSCs) such as methyl mercaptan produced by harmful bacteria or food.

The prebiotic fibre in the first soft capsule as taught herein advantageously optimizes the oral microflora by selectively stimulating the growth and/or activity of beneficial bacteria. Thereby, the prebiotic fibre advantageously allows reducing the proliferation of harmful bacteria.

The active ingredients in the first soft capsule as taught herein allow immediate and long-term effective treatment and/or prevention of halitosis in a subject

In the present soft capsules, the second soft capsule as taught herein comprises an active ingredient capable of blocking adhesion of harmful bacteria to the stomach and an alginate.

The active ingredient capable of blocking adhesion of harmful bacteria to the stomach in the second soft capsule as taught herein advantageously neutralizes harmful bacteria in the stomach. The active ingredient capable of blocking adhesion of harmful bacteria to the stomach adheres to the harmful bacteria such as *Helicobacter pylori* without destroying them, thereby blocking bacterial adhesion to the mucosa of the stomach while advantageously preserving immunogenicity. The natural immune system of the subject can react to the harmful bacteria such as *Helicobacter pylori,* thereby allowing that the subject develops immunity.

The alginate in the second soft capsule as taught herein allows the formation of a protective layer on the top of the stomach, thereby suppressing gastro-oesophageal reflux and preventing the reflux of gastric acids.

The active ingredients in the second soft capsule as taught herein allow immediate and long-term effective treatment and/or prevention of halitosis in a subject.

The phrase "an active ingredient capable of blocking adhesion of harmful bacteria to the oral cavity" includes the wording "one or more active ingredients capable of blocking adhesion of harmful bacteria to the oral cavity".

Accordingly, in certain embodiments, the first soft capsule may consist essentially of or consist of one or more active ingredients capable of blocking adhesion of harmful bacteria to the oral cavity, a zinc salt, and a prebiotic fibre, wherein the one or more active ingredients capable of blocking adhesion of harmful bacteria to the oral cavity are selected from the group consisting of cranberry extract, wild blueberry extract, strawberry extract, tea tree oil, and chamomile oil.

In certain embodiments of the soft capsules or methods, as taught herein, the one or more active ingredients, such as two or more, or three or more, or four or more active ingredients, capable of blocking adhesion of harmful bacteria to the oral cavity may be selected from the group consisting of cranberry extract, wild blueberry extract, strawberry extract, tea tree oil, and chamomile oil.

In certain embodiments of the soft capsules or methods, as taught herein, the one or more active ingredients, such as two or more, or three or more, or four or more active ingredients, capable of blocking adhesion of harmful bacteria to the stomach may be selected from the group consisting of cranberry extract, wild blueberry extract, strawberry extract, tea tree oil, chamomile oil, tea extract, neem stick extract, snowdrop extract, seaweed extract, hop bract extract, Galla chinensis extract, avocado, and legume storage proteins.

In certain embodiments of the soft capsules or methods, as taught herein, the one or more active ingredients capable of blocking adhesion of harmful bacteria to the oral cavity or to the stomach may be selected from the group consisting of cranberry extract, (wild) blueberry extract, or strawberry extract. These extracts advantageously allow neutralizing harmful bacteria in the oral cavity and in the stomach. Such extracts adhere to the harmful bacteria without destroying them, thereby blocking bacterial adhesion to the mouth tissue (e.g., oral mucosa) or stomach tissue (e.g., stomach mucosa) while advantageously allowing immunogenicity.

In certain embodiments of the soft capsules or methods, as taught herein, the one or more active ingredients capable of blocking adhesion of harmful bacteria to the stomach may be selected from the group consisting of cranberry extract, wild blueberry extract, strawberry extract, tea tree oil, and chamomile oil. In certain embodiments, the one or more active ingredients capable of blocking adhesion of harmful bacteria to the oral cavity or to the stomach may be selected from the group consisting of cranberry extract, tea tree oil, and chamomile oil.

In certain embodiments, the one or more active ingredients capable of blocking adhesion of harmful bacteria to the oral cavity or to the stomach may be cranberry extract and tea tree oil. In certain embodiments, the one or more active ingredients capable of blocking adhesion of harmful bacteria to the oral cavity or to the stomach may be cranberry extract and chamomile oil. In certain embodiments, the one or more active ingredients capable of blocking adhesion of harmful bacteria to the oral cavity or to the stomach may be tea tree oil and chamomile oil. In certain embodiments, the one or more active ingredients capable of blocking adhesion of harmful bacteria to the oral cavity or to the stomach may be cranberry extract, tea tree oil, and chamomile oil.

In certain embodiments of the soft capsules or methods, as taught herein, the active ingredient capable of blocking adhesion of harmful bacteria to the oral cavity or to the stomach may be cranberry extract. In certain embodiments, the active ingredient capable of blocking adhesion of harmful bacteria to the oral cavity or to the stomach may be tea tree oil. In certain embodiments, the active ingredient capable of blocking adhesion of harmful bacteria to the oral cavity or to the stomach may be chamomile oil.

In certain embodiments of the soft capsules or methods, as taught herein, the active ingredient capable of blocking adhesion of harmful bacteria to the stomach may be selected from the group consisting of cranberry extract, tea extract, neem stick extract, snowdrop extract, seaweed extract, hop bract extract, Galla chinensis extract, avocado, and legume storage proteins.

In certain embodiments of the soft capsules or methods, as taught herein, the active ingredient capable of blocking adhesion of harmful bacteria to the stomach may be selected from the group consisting of cranberry (*Vaccinium macrocarpon*) extract, green tea (*Camillia sinensis*) extract, oolong tea extract, neem stick (*Azadirachta indica*) extract, snowdrop (*Gilanthus nivalis*) extract, seaweed (*Gloipeltis furcata*) extract, seaweed (*Gigartine teldi*) extract, hop bract or leaves extract, Galla chinensis extract, avocado (*Persea Americana*)*,* and a legume storage protein.

In certain preferred embodiments, the active ingredient capable of blocking adhesion of harmful bacteria to the oral cavity or to the stomach is cranberry extract. Cranberry extract advantageously allows neutralizing harmful bacteria in the oral cavity and in the stomach. Cranberry extract adheres to the harmful bacteria without destroying them, thereby blocking bacterial adhesion to the mouth tissue (e.g., oral mucosa) or stomach tissue (e.g., stomach mucosa) while advantageously allowing immunogenicity.

Cranberry extract is rich in proanthocyanidins (PACs). Proanthocyanidins (PACs), also known as oligomeric proanthocyanidins (OPC), pycnogenol, leukocyanidins, or leucoanthocyanins, are a class of polyphenols found in a variety of plants. Chemically, proanthocyanidins are oligomeric flavonoids.

In certain embodiments, the concentration of the proanthocyanidins in the cranberry extract is from 5 µg/ml to 100 µg/ml.

Cranberry extract is commercially available for example as Exocyan™ Cran 70S (Nexira, Rouen Cedex, France).

In certain embodiments, the active ingredient capable of blocking adhesion of harmful bacteria to the oral cavity or to the stomach may be wild blueberry extract or strawberry extract. Wild blueberry extract and strawberry extract are rich in polyphenols.

In certain embodiments, the active ingredient capable of blocking adhesion of harmful bacteria to the oral cavity or to the stomach may be tea tree (*Melaleuca alternifolia*) oil. Tea tree oil advantageously allows neutralizing harmful bacteria in the oral cavity and in the stomach. Tea tree oil is defined by the International Standard ISO 4730 ("Oil of Melaleuca, Terpinen-4-ol type"), which specifies levels of 15 components which define the oil as "tea tree oil".

In certain embodiments, the active ingredient capable of blocking adhesion of harmful bacteria to the oral cavity or to the stomach may be chamomile oil. Chamomile oil advantageously allows neutralizing harmful bacteria in the oral cavity and in the stomach.

In certain embodiments, the active ingredient capable of blocking adhesion of harmful bacteria to the stomach is tea extract. In certain embodiments, the tea extract may be extract of green tea (*Camillia sinensis*) or extract of oolong tea.

Green tea extract is rich in (-) epicatechin gallate and (-) gallocatechin gallate. Oolong tea extract is rich in polyphenols.

Green tea extract is commercially available for example as green tea extract 95% (Product No. P07361, Cambridge Commodities Ltd., Cambridgeshire, UK) or green tea extract 98% (Product No. P07389, Cambridge Commodities Ltd., Cambridgeshire, UK).

In certain embodiments, the active ingredient capable of blocking adhesion of harmful bacteria to the stomach is Galla chinensis extract.

The term "Galla chinensis extract" as used herein refers to extract of galls produced on the Chinese sumac (*Rhus chinensis*) by the Chinese sumac aphid, also known as *Schlectendalia chinensis* or *Melaphis chinensis.* Galla chinensis extract is rich in gallotannins, a type of hydrolysable tannins.

In certain embodiments, the active ingredient capable of blocking adhesion of harmful bacteria to the stomach is a legume storage protein.

Generally, legume storage proteins are present in seeds, particularly of leguminous plants. Legume storage proteins contain glycoproteins.

In certain embodiments, the first soft capsule may comprise the active ingredient capable of blocking adhesion of harmful bacteria to the oral cavity, such as a cranberry extract, in an amount of 1 to 90 parts by weight based on 100 parts by weight of the first soft capsule. For instance, the first soft capsule may comprise the active ingredient capable of blocking adhesion of harmful bacteria to the oral cavity, such as a cranberry extract, in an amount of 1 to 75 parts by weight, 1 to 50 parts by weight, 1 to 25 parts by weight, 1 to 10 parts by weight, or 1 to 5 parts by weight based on 100 parts by weight of the first soft capsule.

In certain embodiments, the second soft capsule may comprise the active ingredient capable of blocking adhesion of harmful bacteria to the stomach, such as a cranberry extract, in an amount of 1 to 90 parts by weight based on 100 parts by weight of the second soft capsule. For instance, the second soft capsule may comprise the active ingredient capable of blocking adhesion of harmful bacteria to the stomach, such as a cranberry extract, in an amount of 1 to 75 parts by weight, 1 to 50 parts by weight, 1 to 25 parts by weight, or 1 to 10 parts by weight based on 100 parts by weight of the second soft capsule.

In certain embodiments of the soft capsules or methods, as taught herein, the metal salt may be a zinc salt selected from the group consisting of zinc acetate, zinc gluconate, zinc picolinate, zinc amino acid chelate, zinc chloride, zinc citrate, zinc bisglycinate chelate, zinc stearate, zinc lactate, zinc ammonium sulfate, zinc chromate, zinc dithionate, zinc fluorosilicate, zinc tartrate, zinc formate, zinc iodide, zinc nitrate, zinc phenol sulfonate, zinc salicylate, zinc sulfate, zinc succinate, zinc glycerophosphate, and zinc halides. A zinc salt in the first soft capsule as taught herein advantageously allows eliminating volatile malodour gases by capturing VSCs such as methyl mercaptan.

In certain embodiments of the soft capsules or methods, as taught herein, the metal salt may be a zinc salt selected from the group consisting of zinc acetate, zinc gluconate, zinc picolinate, zinc amino acid chelate, zinc chloride, zinc citrate, and zinc bisglycinate chelate.

In certain embodiments of the soft capsules or methods, as taught herein, the metal salt is zinc acetate.

In certain embodiments, the first soft capsule may comprise the zinc salt, such as zinc acetate, in an amount of 0.01 to 10 parts by weight based on 100 parts by weight of the first soft capsule. For instance, the first soft capsule may comprise the zinc salt, such as zinc acetate, in an amount of 0.01 to 5 parts by weight, 0.05 to 5 parts by weight, 0.1 to 2 parts by weight, 0.1 to 1 parts by weight, or 0.1 to 0.5 parts by weight based on 100 parts by weight of the first soft capsule.

In certain embodiments of the soft capsules or methods, as taught herein, the prebiotic fibre may be selected from the group consisting of inulin, oligofructose, lactose, resistant starch, fructooligosaccharides (FOS), galactooligosaccharides (GOS), transgalactooligosaccharides (TOS), xylooligosaccharides (XOS), polydextrose, wheat dextrin, acacia gum, psyllium, banana, whole grain wheat, and whole grain corn.

In certain embodiments of the soft capsules or methods, as taught herein, the prebiotic fibre may be selected from the group consisting of inulin, oligofructose, resistant starch, fructooligosaccharides (FOS), galactooligosaccharides (GOS), transgalactooligosaccharides (TOS), xylooligosaccharides (XOS), polydextrose, wheat dextrin, acacia gum, psyllium, banana, whole grain wheat, and whole grain corn. In certain embodiments of the soft capsules or methods, as taught herein, the prebiotic fibre may be inulin or an oligosaccharide. In certain embodiments, the prebiotic fibre may be inulin or an oligosaccharide selected from the group consisting of FOS, GOS, TOS, and XOS. In certain embodiments of the soft capsules or methods, as taught herein, the prebiotic fibre may be selected from the group consisting of inulin, FOS, GOS, TOS, and XOS.

In certain embodiments of the soft capsules or methods, as taught herein, the prebiotic fibre is inulin.

In certain embodiments, the prebiotic fibre such as inulin may be mixed with a dairy product such as yoghurt. In certain embodiments of the soft capsules or methods, as taught herein, the prebiotic fibre is a blend of inulin and yoghurt. In certain embodiments, the first soft capsule comprises a blend of inulin and yoghurt.

A blend of inulin and yoghurt is commercially available as Yoghurtene Balance CQ with product number 6099946 (Givaudan UK Ltd., Ashford, UK). Yoghurtene contains whey proteins, lactose, lactic acid, vitamins A, vitamins B, and minerals. Yoghurtene has low fat content. Yoghurtene does not contain casein proteins.

In certain embodiments, the first soft capsule may comprise the prebiotic fibre, such as a blend of inulin and yoghurt, in an amount of 0.01 to 10 parts by weight based on 100 parts by weight of the first soft capsule. For instance, the first soft capsule may comprise the prebiotic fibre, such as a blend of inulin and yoghurt, in an amount of 0.01 to 5 parts by weight, 0.05 to 5 parts by weight, 0.1 to 2 parts by weight, 0.1 to 1 parts by weight, or 0.5 to 1 parts by weight based on 100 parts by weight of the first soft capsule.

In certain embodiments of the soft capsules or methods, as taught herein, the first soft capsule may comprise a cranberry extract, a zinc salt, and a prebiotic fibre. In certain embodiments, the first soft capsule may comprise a cranberry extract, a zinc acetate, and a blend of inulin and yoghurt.

The phrase "an active ingredient capable of blocking adhesion of harmful bacteria to the stomach" as used herein includes the wording "one or more active ingredients capable of blocking adhesion of harmful bacteria to the stomach".

Hence, in certain embodiments, the second soft capsule may consist essentially of or consist of one or more active ingredients capable of blocking adhesion of harmful bacteria to the stomach and an alginate, wherein the one or more active ingredients capable of blocking adhesion of harmful bacteria to the stomach are selected from the group consisting of cranberry extract, wild blueberry extract, strawberry extract, tea tree oil, chamomile oil, tea extract, neem stick extract, snowdrop extract, seaweed extract, hop bract extract, Galla chinensis extract, avocado, and legume storage proteins.

In certain embodiments of the soft capsules or methods, as taught herein, the alginate may be selected from the group consisting of sodium alginate, potassium alginate, and calcium alginate.

In certain embodiments, the alginate is sodium alginate. Sodium alginate is a gum, extracted from the cell walls of brown algae.

In certain embodiments, the second soft capsule may comprise the alginate, such as sodium alginate, in an amount of 1 to 50 parts by weight based on 100 parts by weight of the second soft capsule. For instance, the second soft capsule may comprise the alginate, such as sodium alginate, in an amount of 1 to 25 parts by weight, 1 to 10 parts by weight, or 1 to 5 parts by weight based on 100 parts by weight of the second soft capsule

In certain embodiments of the soft capsules or methods, as taught herein, the second soft capsule may comprise a cranberry extract and an alginate. In certain embodiments, the second soft capsule may comprise a cranberry extract and sodium alginate.

In certain embodiments, the first soft capsule may further comprise an edible vegetable oil. The edible vegetable oil allows to control the flavour and/or to improve the stability of the first soft capsule as taught herein and/or functions as a solvent and/or functions as a carrier. The vegetable oil as defined herein may be one or more of tea tree oil, palm oil, sunflower oil, safflower oil, sesame oil, rapeseed oil, and grape-seed oil. In certain embodiments, the first soft capsule may comprise the edible vegetable oil in an amount of 0 to 99 parts by weight based on 100 parts by weight of the first soft capsule.

In certain embodiments, the first soft capsule may comprise an active ingredient capable of blocking adhesion of harmful bacteria to the oral cavity, a zinc salt, a prebiotic fibre, and an edible vegetable oil.

In certain embodiments, the first soft capsule may also comprise a flavouring component. The flavouring component may be mint flavour, spearmint flavour, strawberry flavour, vanilla flavour, cinnamon flavour, and/or citrus flavour. In certain embodiments, the first soft capsule may comprise the flavouring component in an amount of 0.01 to 50 parts by weight based on 100 parts by weight of the first soft capsule.

In certain embodiments, the first soft capsule may comprise an active ingredient capable of blocking adhesion of harmful bacteria to the oral cavity, a zinc salt, a prebiotic fibre, and a flavouring component. In certain embodiments, the first soft capsule may comprise an active ingredient capable of blocking adhesion of harmful bacteria to the oral cavity, a zinc salt, a prebiotic fibre, an edible vegetable oil, and a flavouring component.

The flavouring component may be a sweetening, an acidulant, and a bitter flavouring. An example of the sweetening may be aspartame, stevia, saccharin sodium, or thaumatin. An example of the acidulant may be citric acid, malic acid, tartaric acid, or fumaric acid. An example of the bitter flavouring may be caffeine or naringin. In certain embodiments, the first soft capsule may comprise at least one flavouring component selected from the sweetening, the acidulant, and the bitter flavouring.

In certain embodiments, the first soft capsule may further comprise a colorant and/or an aroma. The colorant and aroma may be as known in the art. In certain embodiments, the first soft capsule may comprise the colorant and/or the aroma in an amount not more than 80 parts by weight based on 100 parts by weight of the first soft capsule.

In certain embodiments, the first soft capsule may comprise an active ingredient capable of blocking adhesion of harmful bacteria to the oral cavity, a zinc salt, a prebiotic fibre, and a colorant and/or an aroma. In certain embodiments, the first soft capsule may comprise an active ingredient capable of blocking adhesion of harmful bacteria to the oral cavity, a zinc salt, a prebiotic fibre, an edible vegetable oil, a flavouring component, and a colorant and/or an aroma. In certain embodiments, the first soft capsule may comprise an active ingredient capable of blocking adhesion of harmful bacteria to the oral cavity, a zinc salt, a prebiotic fibre, and optionally an edible vegetable oil, a flavouring component, a colorant, and/or an aroma.

In certain embodiments, the second soft capsule may further comprise an edible vegetable oil as defined herein. The edible vegetable oil allows to control the flavour and/or to improve the stability of the second soft capsule as taught herein and/or functions as a solvent and/or functions as a carrier. In certain embodiments, the second soft capsule may comprise the edible vegetable oil in an amount of 0 to 99 parts by weight based on 100 parts by weight of the second soft capsule.

Hence, in certain embodiments, the second soft capsule may comprises an active ingredient capable of blocking adhesion of harmful bacteria to the stomach, an alginate, and an edible vegetable oil.

In certain embodiments, the second soft capsule may further comprise a colorant and/or an aroma, as defined herein. A total amount of the colorant and/or the aroma formulated in the second soft capsule may preferably be not more than 80 parts by weight based on 100 parts by weight of the second soft capsule.

Accordingly, in certain embodiments, the second soft capsule may comprises an active ingredient capable of blocking adhesion of harmful bacteria to the stomach, an alginate, and a colorant and/or an aroma. In certain embodiments, the second soft capsule may comprises an active ingredient capable of blocking adhesion of harmful bacteria to the stomach, an alginate, an edible vegetable oil, and a colorant and/or an aroma. In certain embodiments, the second soft capsule may comprises an active ingredient capable of blocking adhesion of harmful bacteria to the stomach, an alginate, and optionally an edible vegetable oil, a colorant, and/or an aroma.

In certain embodiments of the soft capsules or methods, as taught herein, the first and/or second soft capsule may further comprise one or more pharmaceutical excipients. Suitable pharmaceutical excipients depend on the dosage form and identities of the active ingredients and can be selected by the skilled person (e.g. by reference to the Handbook of Pharmaceutical Excipients 6th Edition 2009, eds. Rowe et al.). As used herein, "carrier" or "excipient" includes any and all solvents, diluents, buffers (such as neutral buffered saline or phosphate buffered saline), solubilisers, colloids, dispersion media, vehicles, fillers, chelating agents (such as EDTA or glutathione), amino acids (such as glycine), proteins, disintegrants, binders, lubricants, wetting agents, emulsifiers, sweeteners, colorants, flavourings, aromatisers, thickeners, agents for achieving a depot effect, coatings, antifungal agents, preservatives, stabilisers, antioxidants, tonicity controlling agents, absorption delaying agents, and the like. The use of such media and agents for pharmaceutical active substances is well known in the art. Such materials should be non-toxic and should not interfere with the activity of the active ingredients.

In certain embodiments of the soft capsules or methods, as taught herein, the first and/or second soft capsule may comprise pharmaceutically acceptable auxiliary substances as required to approximate physiological conditions, such as pH adjusting and buffering agents, preservatives, complexing agents, tonicity adjusting agents, wetting agents and the like, for example, sodium acetate, sodium lactate, sodium phosphate, sodium hydroxide, hydrogen chloride, benzyl alcohol, parabens, EDTA, sodium oleate, sodium chloride, potassium chloride, calcium chloride, sorbitan monolaurate, triethanolamine oleate, etc.

In certain embodiments of the soft capsules or methods, as taught herein, the first and/or second soft capsule may further comprise one or more compounds selected from the group consisting of plant extracts, vitamins, minerals, amino acids, and enzymes.

In certain embodiments, the weight of the first soft capsule or outer capsule is from 100 mg to 300 mg. For example, the weight of the first soft capsule or outer capsule is from 200 mg to 300 mg, from 220 mg to 300 mg, from 220 mg to 280 mg, from 230 mg to 290 mg, or from 235 mg to 287 mg. In certain embodiments, the weight of the first soft capsule or outer capsule is about 261 mg (e.g., 261 mg ± 26 mg).

The recitations "weight of the first soft capsule", "weight of the outer capsule", "first soft capsule weight", or "outer capsule weight" can be used interchangeably and refers to the (total) weight of the soft capsule.

The term "weight" as used herein refers to the average weight. The average weight can be determined by weighing 20 capsules or beads with an electric balance and dividing the result by 20. Typically, every capsule or bead is weighted 3 times.

In certain embodiments, the first soft capsule may comprise from 1 mg to 150 mg by weight of the cranberry extract, from 1 mg to 10 mg by weight of zinc salt, and from 1mg to 10 mg by weight of prebiotic fibre. In certain embodiments, the first soft capsule may comprises from 1 mg to 150 mg by weight of cranberry extract, from 1 mg to 10 mg by weight of zinc acetate, and from 1mg to 10 mg by weight of a blend of inulin and yoghurt.

In certain embodiments, the weight of the second soft capsule or inner capsule is from 5 mg to 50 mg. For example, the weight of the second soft capsule or outer capsule is from 10 mg to 40 mg, from 20 mg to 40 mg, from 25 mg to 45 mg, from 25 mg to 40 mg, from 25 mg to 35 mg, from 30 mg to 40 mg, or from 30mg to 38 mg. In certain embodiments, the weight of the second soft capsule or inner capsule is about 34 mg (e.g., 34 mg ± 4 mg).

The recitations "weight of the second soft capsule", "weight of the inner capsule", "second soft capsule weight", or "inner capsule weight" can be used interchangeably and refers to the (total) weight of the second soft capsule.

In certain embodiments, the second soft capsule may comprise from 1 mg to 15 mg by weight of the cranberry extract and from 1 mg to 15 mg by weight of the alginate.

In certain embodiments of the soft capsules and methods, as taught herein, the diameter of the first soft capsule may be from 2.0 mm to 12.0 mm. For example, the diameter of the first soft capsule may be from 4.0 mm to 10.0 mm or from 6.0 mm to 10.0 mm. In certain embodiments, the diameter of the first soft capsule is about 8.0 mm (e.g., 8.0 mm ± 0.2 mm).

The recitations "diameter of the first soft capsule", "diameter of the outer capsule", "first soft capsule diameter", or "outer capsule diameter" can be used interchangeably and refers to the diameter of the soft capsule.

The term "diameter" as used herein refers to the average diameter. The average diameter can be determined by measuring 20 capsules or beads in line with ruler and dividing the result by 20. Typically, every capsule or bead is measured 3 times.

In certain embodiments of the soft capsules and methods, as taught herein, the diameter of the second soft capsule may be from 0.5 mm to 6.0 mm. For example, the diameter of the second soft capsule may be from 1.0 mm to 6.0 mm, from 2.0 to 6.0 mm, or from 3.0 to 5.0 mm. In certain embodiments, the diameter of the second soft capsule is about 4.0 mm (e.g., 4.0 mm ± 0.2 mm).

The recitations "diameter of the second soft capsule", "diameter of the inner capsule", "second soft capsule diameter", or "inner capsule diameter" can be used interchangeably and refers to the diameter of the second soft capsule.

In certain embodiments, the soft capsule as taught herein may comprise a first soft capsule composed of a first soft capsule layer and a first soft capsule content, and a second soft capsule composed of a second soft capsule layer and a second soft capsule content.

In certain embodiments, the soft capsule as taught herein is a multi-layered soft capsule or capsule-in-capsule comprising a first soft capsule composed of a first soft capsule layer and a first soft capsule content, and a second soft capsule composed of a second soft capsule layer and a second soft capsule content. Advantageously, the multi-layered structure or capsule-in-capsule structure of the soft capsule as taught herein allows a first release in the oral cavity of the active ingredients having an effect in the oral cavity followed by a second release in the stomach of the active ingredients having an effect in the stomach. The release in the oral cavity of the active ingredients having an effect in the oral cavity following by a release in the stomach of the active ingredients having an effect in the stomach allows treating halitosis effectively both instantly and in the long term.

In certain embodiments, the soft capsule as taught herein comprises or consists of four layers. The four layers can be referred to, from the outside to the inside of the capsule, as a first soft capsule layer (or outer layer), a first soft capsule content (or outer content), second soft capsule layer (or inner layer), and second soft capsule content (or inner content).

In certain embodiments, the soft capsule as taught herein comprises a first soft capsule and a second soft capsule encapsulated in the first soft capsule. In certain embodiments, the first soft capsule is composed of a first soft capsule layer and a first soft capsule content containing the active ingredients having an effect in the oral cavity, wherein the first soft capsule content is encapsulated in the first soft capsule layer. In certain embodiments, the second soft capsule is composed of a second soft capsule layer and a first soft capsule content containing the active ingredients having an effect in the stomach, wherein the second soft capsule content is encapsulated in the second soft capsule layer.

The recitation "active ingredients having an effect in the oral cavity" as used herein refers to (the combination of) at least an active ingredient capable of blocking adhesion of harmful bacteria to the oral cavity, a zinc salt, and a prebiotic fibre.

The recitation "active ingredients having an effect in the stomach" as used herein refers to (the combination of) at least an active ingredient capable of blocking adhesion of harmful bacteria to the stomach and an alginate.

In certain embodiments, the first soft capsule layer comprises a zinc salt, preferably zinc acetate.

In certain embodiments, the first soft capsule layer comprises a zinc salt, preferably zinc acetate; and a base material such as gelatine, agar, methylcellulose, hypromellose or hydroxypropylmethylcellulose (HPMC), or a mixture of any two or more thereof, preferably gelatine. In certain embodiments, the first soft capsule layer comprises a zinc salt and gelatine. In certain preferred embodiments, the first soft capsule layer comprises zinc acetate and gelatine.

In certain embodiments, the second soft capsule layer comprises an active ingredient capable of blocking adhesion of harmful bacteria to the stomach, preferably a cranberry extract, and an alginate, preferably sodium alginate.

In certain embodiments, the second soft capsule layer comprises an active ingredient capable of blocking adhesion of harmful bacteria to the stomach, an alginate; and a base material such as gelatine, agar, methylcellulose, hypromellose or HPMC, or a mixture of any two or more thereof, preferably gelatine. In certain embodiments, the second soft capsule layer comprises a cranberry extract, sodium alginate, and a base material such as gelatine, agar, methylcellulose, hypromellose or HPMC, or a mixture of any two or more thereof, preferably gelatine. In certain embodiments, the second soft capsule layer comprises a cranberry extract, sodium alginate, and gelatine.

In certain embodiments, the first and second soft capsule layer may comprise a base material. The base material for forming the first and second soft capsule layer may be any material capable of forming a soft capsule layer such as gelatine, agar, methylcellulose, hypromellose or HPMC, or a mixture of any two or more thereof.

In addition to the base material mentioned above, the material for forming the first and second soft capsule layers of a multi-layered soft capsule as taught herein may also comprise a water-soluble polyol such as sorbitol, glycerine and the like, sodium alginate or low methoxyl pectin or one obtained by gelling an aqueous solution of sodium alginate or low methoxyl pectin with a polyvalent ion, preferably bi- or more valent ion or the like. The polyvalent ion can be a salt of polyvalent metal such as bi- or more valent metal, particularly when gelling the aqueous solution of sodium alginate, a water-soluble calcium salt, such as calcium chlorate, calcium phosphate and the like is suitably used, or when gelling low methoxyl pectin, a water-soluble salt, such as calcium, magnesium or the like is suitably used.

In certain embodiments, the material for forming the first and second soft capsule layers may further contain a flavouring component as defined herein.

The flavouring component may be a sweetening, an acidulant, and a bitter flavouring. An example of the sweetening may be aspartame, stevia, saccharin sodium, or thaumatin. An example of the acidulant may be citric acid, malic acid, tartaric acid, or fumaric acid. An example of the bitter flavouring may be caffeine or naringin. In certain embodiments, the first soft capsule layer and/or the second soft capsule layer may comprise at least one flavouring component selected from the sweetening, the acidulant, and the bitter flavouring.

In certain embodiments, the material for forming the first and second soft capsule layers of the soft capsules as taught herein may further comprise an adjuvant selected from the group consisting of a plasticizer, a preservative, a colorant, and an aroma.

In certain embodiments, the total amount of the adjuvant and the flavouring component formulated in the material for forming the first and second soft capsule layers may be 0.1 to 50 parts by weight, based on 100 parts by weight of the material for forming the first and second soft capsule layers. Preferably, the flavouring component may be in an amount of 0.1 to 30 parts by weight based on 100 parts by weight of the material.

In order to make the second soft capsule insoluble in an oral cavity and soluble in a stomach, the material for forming the second soft capsule layers may further comprise a substance such as agar or gum Arabic. In certain embodiments, the second soft capsule layers may further comprise a substance such as agar or gum Arabic in an amount of 0.01 to 10% by weight based on the total amount of the adjuvant.

In certain embodiments, the first soft capsule content may comprise an active ingredient capable of blocking adhesion of harmful bacteria to the oral cavity and a prebiotic fibre.

In certain embodiments, the first soft capsule content may further comprise an edible vegetable oil as defined herein. The edible vegetable oil allows to control the flavour and to improve the stability of the soft capsule as taught herein and/or functions as a solvent and/or functions as a carrier. In certain embodiments, the first soft capsule content may comprise the edible vegetable oil in an amount of 0 to 99 parts by weight based on 100 parts by weight of the content.

In certain embodiments, the first soft capsule content may also comprise a flavouring component as described herein. In certain embodiments, the first soft capsule content may comprise the flavouring component in an amount of 0.01 to 50 parts by weight based on 100 parts by weight of the first soft capsule content.

In certain embodiments, the first soft capsule content may further comprise a colorant and/or an aroma, as defined herein. In certain embodiments, the first soft capsule content may comprise the colorant and/or the aroma in an amount not more than 80 parts by weight based on 100 parts by weight of the first soft capsule content.

In certain embodiments, the second soft capsule content may comprise, consist essentially of, or consist of an active ingredient capable of blocking adhesion of harmful bacteria to the stomach.

In certain embodiments, the second soft capsule content may also comprise an edible vegetable oil, a colorant, and/or an aroma, as defined herein. In certain embodiments, the second soft capsule content may comprise the edible vegetable oil in an amount of 0 to 99 parts by weight based on 100 parts by weight of the second soft capsule content. A total amount of the colorant and/or the aroma formulated in the second soft capsule content may preferably be not more than 80 parts by weight based on 100 parts by weight of the second soft capsule content.

In certain embodiments, a total amount of the first and second soft capsule contents may be 10% to 95% by weight, preferably 50% to 90% by weight, based on the total weight of the soft capsule.

In certain embodiments of the soft capsules or methods, as taught herein, the soft capsule is configured for oral administration.

In certain embodiments, the soft capsules as taught herein may be gelatine capsules (i.e., capsulae gelatinosae), also referred to as gel caps or gelcaps. In certain embodiments, the soft capsules as taught herein may be vegetable or vegetarian capsules. Examples of vegetarian capsules are Vegicaps® (Catalent Pharma Solutions, Somerset, USA) or Vcaps® Plus capsules (Capsugel, Bornem, Belgium).

The soft capsules as taught herein may advantageously be used as dietary supplements or medical devices. A further aspect relates to the use of the soft capsules as taught herein as a dietary supplement or a medical device.

A further aspect relates to the soft capsules as taught herein for use as a medicament. In other words, a further aspect relates to the soft capsules as taught herein for use as a medicinal product.

In certain embodiments, the present invention provides the soft capsules as taught herein for use in the treatment (including throughout the present specification therapeutic and/or preventative measures) of halitosis.

The present invention provides a soft capsule for use in the treatment of halitosis, wherein the soft capsule comprises (a) a first soft capsule configured for targeted delivery to the mouth and (b) a second soft capsule configured for targeted delivery to the stomach, wherein the first soft capsule comprises one or more active ingredients capable of blocking adhesion of harmful bacteria to the oral cavity, a zinc salt, and a prebiotic fibre, wherein the one or more active ingredients capable of blocking adhesion of harmful bacteria to the oral cavity are selected from the group consisting of cranberry extract, wild blueberry extract, strawberry extract, tea tree oil, and chamomile oil; the second soft capsule comprises one or more active ingredients capable of blocking adhesion of harmful bacteria to the stomach and an alginate, wherein the one or more active ingredients capable of blocking adhesion of harmful bacteria to the stomach are selected from the group consisting of cranberry extract, wild blueberry extract, strawberry extract, tea tree oil, chamomile oil, tea extract, neem stick extract, snowdrop extract, seaweed extract, hop bract extract, Galla chinensis extract, avocado, and legume storage proteins; and the second soft capsule is contained in the first soft capsule. The use of the soft capsules as taught herein in the treatment of halitosis is advantageous *inter alia* because these soft capsules allow efficient treatment during longer periods due to the combined action of the active ingredients in the mouth and in the stomach. A further advantage of the present soft capsules is that they also have an instant effect without any side effects.

Also provided is the use of a soft capsule for the manufacture of a medicament for the treatment of halitosis, wherein the soft capsule comprises (a) a first soft capsule configured for targeted delivery to the mouth and (b) a second soft capsule configured for targeted delivery to the stomach, wherein the first soft capsule comprises one or more active ingredients capable of blocking adhesion of harmful bacteria to the oral cavity, a zinc salt, and a prebiotic fibre, wherein the one or more active ingredients capable of blocking adhesion of harmful bacteria to the oral cavity are selected from the group consisting of cranberry extract, wild blueberry extract, strawberry extract, tea tree oil, and chamomile oil; the second soft capsule comprises one or more active ingredients capable of blocking adhesion of harmful bacteria to the stomach and an alginate, wherein the one or more active ingredients capable of blocking adhesion of harmful bacteria to the stomach are selected from the group consisting of cranberry extract, wild blueberry extract, strawberry extract, tea tree oil, chamomile oil, tea extract, neem stick extract, snowdrop extract, seaweed extract, hop bract extract, Galla chinensis extract, avocado, and legume storage proteins; and the second soft capsule is contained in the first soft capsule. Such treatment typically involves oral administration of the soft capsules.

Also provided is a method for treating halitosis in a subject in need of such treatment, comprising administering to said subject a therapeutically or prophylactically effective amount of a soft capsule comprising (a) a first soft capsule configured for targeted delivery to the mouth and (b) a second soft capsule configured for targeted delivery to the stomach, wherein the first soft capsule comprises one or more active ingredients capable of blocking adhesion of harmful bacteria to the oral cavity, a zinc salt, and a prebiotic fibre, wherein the one or more active ingredients capable of blocking adhesion of harmful bacteria to the oral cavity are selected from the group consisting of cranberry extract, wild blueberry extract, strawberry extract, tea tree oil, and chamomile oil; the second soft capsule comprises one or more active ingredients capable of blocking adhesion of harmful bacteria to the stomach and an alginate, wherein the one or more active ingredients capable of blocking adhesion of harmful bacteria to the stomach are selected from the group consisting of cranberry extract, wild blueberry extract, strawberry extract, tea tree oil, chamomile oil, tea extract, neem stick extract, snowdrop extract, seaweed extract, hop bract extract, Galla chinensis extract, avocado, and legume storage proteins; and the second soft capsule is contained in the first soft capsule.

Such method of treatment typically involves oral administration of the soft capsules.

The terms "halitosis", "bad breath", "oral malodour", "breath malodour", "feter oris", or "fege bosta" can be used interchangeably and refer to a disorder in which a noticeably unpleasant odour is present on the exhaled breath.

Two main classification schemes exist for halitosis: the Miyazaki et al. classification (Miyazaki et al., 1999, Niigata Dental Journal, 32, 7-11) and the Tangerman and Winkel classification (Tangerman and Winkel, 2010, Journal of breath research, 4 (1)).

An etiologic classification also exists defining halitosis as Type 0 (physiologic), Type 1 (oral), Type 2 (airway), Type 3 (gastroesophageal), Type 4 (blood-borne), and/or Type 5 (subjective). Any halitosis symptom is potentially the sum of these types in any combination, superimposed on the physiologic odour present in all healthy individuals.

In certain embodiments, the treatment of halitosis comprises an oral treatment of halitosis and a gastrointestinal treatment of halitosis. Advantageously, the combined treatment of the oral cavity and the gastrointestinal tract, in particular the stomach, allows an effective treatment of halitosis with an immediate effect after administration of the soft capsules as taught herein and a long-term effect.

The recitation "oral treatment of halitosis" as used herein refers to a treatment of halitosis in the oral cavity.

The recitation "gastrointestinal treatment of halitosis" as used herein refers to a treatment of halitosis in the gastrointestinal tract, in particular in the stomach.

In certain embodiments of the soft capsules as taught herein, the gastrointestinal treatment of halitosis may comprise a treatment of halitosis in the stomach. As explained herein, the active ingredients having an effect in the stomach advantageously neutralize harmful bacteria in the stomach by blocking bacterial adhesion to the mucosa of the stomach and form a protective layer on the content of the stomach, thereby advantageously resulting in a long-term treatment of halitosis.

Except when noted, the terms "subject" or "patient" can be used interchangeably and refer to animals, preferably warm-blooded animals, more preferably vertebrates, even more preferably mammals, still more preferably primates, and specifically includes human patients and non-human mammals and primates. Preferred subjects are human subjects.

The term "mammal" includes any animal classified as such, including, but not limited to, humans, domestic and farm animals, zoo animals, sport animals, pet animals, companion animals and experimental animals, such as, for example, mice, rats, hamsters, rabbits, dogs, cats, guinea pigs, cattle, cows, sheep, horses, pigs and primates, e.g., monkeys and apes. Particularly preferred are human subjects, including both genders and all age categories thereof.

As used herein, a phrase such as "a subject in need of treatment" includes subjects that would benefit from treatment of a given condition, particularly halitosis. Such subjects may include, without limitation, those that have been diagnosed with said condition, those prone to develop said condition and/or those in who said condition is to be prevented.

The terms "treat" or "treatment" encompass both the therapeutic treatment of an already developed disease or condition, such as the therapy of an already developed halitosis, as well as prophylactic or preventive measures, wherein the aim is to prevent or lessen the chances of incidence of an undesired affliction, such as to prevent occurrence, development and progression of halitosis. Beneficial or desired clinical results may include, without limitation, alleviation of one or more symptoms, diminishment of extent of disease, and the like.

The term "prophylactically effective amount" refers to an amount of an active ingredient or pharmaceutical agent that inhibits or delays in a subject the onset of a disorder as being sought by a researcher, veterinarian, medical doctor or other clinician. The term "therapeutically effective amount" as used herein, refers to an amount of active ingredient or pharmaceutical agent that elicits the biological or medicinal response in a subject that is being sought by a researcher, veterinarian, medical doctor or other clinician, which may include *inter alia* alleviation of the symptoms of the disease or condition being treated. Methods are known in the art for determining therapeutically and prophylactically effective doses for the active ingredients as taught herein.

The invention provides a method for preparing a soft capsule as taught herein, comprising simultaneously extruding a composite jet stream into a cooling solution, wherein the composite jet stream consists of a second soft capsule filling solution extruded through a first nozzle, a second soft capsule layer-forming solution though a second nozzle, a first soft capsule filling solution extruded through a third nozzle and a first soft capsule layer-forming solution extruded through a fourth nozzle, with the first, second, third and fourth nozzles being arranged concentrically, a diameter of the nozzles gradually increasing in a numerical order, and wherein the first soft capsule layer-forming solution comprises a metal salt, wherein the metal salt is a zinc salt; the first soft capsule filling solution comprises one or more active ingredients capable of blocking adhesion of harmful bacteria to the oral cavity and a prebiotic fibre, wherein the one or more active ingredients capable of blocking adhesion of harmful bacteria to the oral cavity are selected from the group consisting of cranberry extract, wild blueberry extract, strawberry extract, tea tree oil, and chamomile oil; the second soft capsule layer-forming solution comprises an alginate; and the second soft capsule filling solution comprises one or more active ingredients capable of blocking adhesion of harmful bacteria to the stomach, wherein the one or more active ingredients capable of blocking adhesion of harmful bacteria to the stomach are selected from the group consisting of cranberry extract, wild blueberry extract, strawberry extract, tea tree oil, chamomile oil, tea extract, neem stick extract, snowdrop extract, seaweed extract, hop bract extract, Galla chinensis extract, avocado, and legume storage proteins. Such method allows producing a seamless soft capsule. An example of such a method is described in US Patent No. US 6,426,089 B1.

In certain embodiments, the methods as taught herein may comprise the prior step of preparing the first soft capsule layer-forming solution by mixing a zinc salt, preferably zinc acetate; and a base material such as gelatine, agar, methylcellulose, HPMC, or a mixture of any two or more thereof, preferably gelatine. In certain embodiments, the methods as taught herein may comprise the prior step of preparing the first soft capsule layer-forming solution by mixing a zinc salt and gelatine. In certain preferred embodiments, the methods as taught herein may comprise the prior step of preparing the first soft capsule layer-forming solution by mixing zinc acetate and gelatine.

In certain embodiments, the methods as taught herein may comprise the prior step of preparing the first soft capsule filling solution by mixing an active ingredient capable of blocking adhesion of harmful bacteria to the oral cavity and a prebiotic fibre. In certain embodiments, the methods as taught herein may comprise the prior step of preparing the first soft capsule filling solution by mixing an active ingredient capable of blocking adhesion of harmful bacteria to the oral cavity, a prebiotic fibre, and an edible vegetable oil. In certain embodiments, the methods as taught herein may further comprise the prior step of preparing the first soft capsule filling solution by mixing an active ingredient capable of blocking adhesion of harmful bacteria to the oral cavity, a prebiotic fibre, an edible vegetable oil, and a flavouring component. In certain embodiments, the methods as taught herein may further comprise the prior step of preparing the first soft capsule filling solution by mixing an active ingredient capable of blocking adhesion of harmful bacteria to the oral cavity, a prebiotic fibre, an edible vegetable oil, a flavouring component, and a colorant and/or an aroma. In certain embodiments, the methods as taught herein may further comprise the prior step of preparing the first soft capsule filling solution by mixing an active ingredient capable of blocking adhesion of harmful bacteria to the oral cavity, a prebiotic fibre, and optionally an edible vegetable oil, a flavouring component, a colorant, and/or aroma.

In certain embodiments, the methods as taught herein may comprise the prior step of preparing the second soft capsule layer-forming solution by mixing an active ingredient capable of blocking adhesion of harmful bacteria to the stomach, an alginate, and a base material such as gelatine, agar, methylcellulose, hypromellose or HPMC, or a mixture of any two or more thereof. In certain embodiments, the methods as taught herein may comprise the prior step of preparing the second soft capsule layer-forming solution by mixing a cranberry extract, sodium alginate, and a base material such as gelatine, agar, methylcellulose, hypromellose or HPMC, or a mixture of any two or more thereof, preferably gelatine. In certain embodiments, the methods as taught herein may comprise the prior step of preparing the second soft capsule layer-forming solution by mixing a cranberry extract, sodium alginate, and gelatine.

In certain embodiments, the methods as taught herein may comprise the prior step of preparing the second soft capsule filling solution by mixing an active ingredient capable of blocking adhesion of harmful bacteria to the stomach, and an edible vegetable oil. In certain embodiments, the methods as taught herein may comprise the prior step of preparing the second soft capsule filling solution by mixing an active ingredient capable of blocking adhesion of harmful bacteria to the stomach, an edible vegetable oil, and a colorant and/or an aroma. In certain embodiments, the methods as taught herein may comprise the prior step of preparing the second soft capsule filling solution by mixing an active ingredient capable of blocking adhesion of harmful bacteria to the stomach, and optionally an edible vegetable oil, a colorant, and/or an aroma.

The soft capsule as taught herein as a final product may be obtained by drying the resulting capsule produced by the above method.

Alternatively, a method for preparing or producing a soft capsule as taught herein, such as a soft capsule for treating halitosis, may comprise preparing a sheet for the second soft capsule layer comprising a material capable of forming a soft capsule layer as the base, an active ingredient capable of blocking adhesion of harmful bacteria to the stomach, and an alginate, and drying to obtain a dried sheet; encapsulating the second soft capsule content comprising an active ingredient capable of blocking adhesion of harmful bacteria to the stomach with the dried sheet on a rotary filler to form the second soft capsule; and on a rotary filler, simultaneously encapsulating both the second soft capsule and the first soft capsule comprising an active ingredient capable of blocking adhesion of harmful bacteria to the oral cavity, and a prebiotic fibre with a sheet for the first soft capsule layer preliminary prepared and dried, wherein the sheet for the first soft capsule comprises a material capable of forming a soft capsule layer as the base and a metal salt, wherein the active ingredient capable of blocking adhesion of harmful bacteria to the oral cavity is selected from the group consisting of cranberry extract, wild blueberry extract, strawberry extract, tea tree oil, and chamomile oil, wherein the metal salt is a zinc salt, and wherein the active ingredient capable of blocking adhesion of harmful bacteria to the stomach is selected from the group consisting of cranberry extract, wild blueberry extract, strawberry extract, tea tree oil, chamomile oil, tea extract, neem stick extract, snowdrop extract, seaweed extract, hop bract extract, Galla chinensis extract, avocado, and legume storage proteins. Such method allows producing a seamed soft capsule. The material capable of forming a soft capsule layer may be gelatine, agar, methylcellulose, hypromellose or hydroxypropylmethylcellulose (HPMC), or a mixture of any two or more thereof.

In certain embodiments of the methods as taught herein, in case the all of the loading materials are liquid, the encapsulation process may be easily performed by adequately vibrating the composite jet stream with a vibration mean to readily release the jet stream, and thereby a particle size of the resulting capsules may be controlled uniformly.

In certain embodiments of the soft capsules and methods, as taught herein, the first soft capsule may be formed into a particle size in a diameter of 2.0 to 12 mm, preferably 8 mm.

In certain embodiments of the soft capsules and methods, as taught herein, the second soft capsule may be formed into a particle size in diameter of 0.5 mm to 6 mm, preferably 4 mm.

The above aspects and embodiments are further supported by the following non-limiting examples.

### EXAMPLES

### Example 1: Soft capsule according to an embodiment of the present invention

The composition of a soft capsule according to an embodiment of the present invention is given in Table 1. Table 1 lists the ingredients and the amount of each ingredient in a first soft capsule according to an embodiment of the present invention. Table 2 lists the ingredients and the amount of each ingredient in a second soft capsule according to an embodiment of the present invention.

**Table 1: First soft capsule (i.e., outer capsule) according to an embodiment of the present invention**

| **Ingredients** | **Commercial name** | **Supplier** | **Amount** |
|---|---|---|---|
| ***First soft capsule layer*** | | | |
| Zinc acetate | Zinc acetate dihydrate | Fagron | 1 mg |
| Porcine gelatine | Gelatine (Pharma) | Gelita | Ad 18 mg |

| ***First soft capsule content*** | | | |
|---|---|---|---|
| Cranberry extract | Exocyan Cran 70S | Nexira | 6.3 mg |
| Prebiotic fibre | Yogurtene | Givaudan | 2 mg |
| Mint flavor | Peppermint Flavouring | Flandor Flavours International | 2 mg |
| Tea tree oil | Tea tree oil - ultra refined | Hallstar Company | Ad 261mg |

**Table 2: Second soft capsule (i.e., inner capsule) according to an embodiment of the present invention**

| **Ingredients** | **Commercial name** | **Supplier** | **Amount** |
|---|---|---|---|
| ***Second soft capsule layer*** | | | |
| Sodium alginate | Protanal LFR5-60 | FMC Biopolymer | 1.6 mg |
| Cranberry extract | Exocyan Cran 70S | Nexira | 0.8 mg |
| Porcine gelatine | Gelatine (Pharma) | Gelita | Ad 8 mg |

| ***Second soft capsule content*** | | | |
|---|---|---|---|
| Cranberry extract | Exocyan Cran 70S | Nexira | 2.6 mg |
| Tea tree oil | Tea tree oil - ultra refined | Hallstar Company | Ad 34 mg |

A soft capsule according to an embodiment of the present invention can be obtained as follows.

A first soft capsule layer-forming solution and a second soft capsule layer-forming solution are prepared for the first soft capsule layer and the second soft capsule layer, respectively. The first soft capsule layer which is a fast dissolve shell comprises zinc acetate and porcine gelatine. The first soft capsule layer-forming solution is prepared by mixing zinc acetate and porcine gelatine. The second soft capsule layer comprises cranberry extract, sodium alginate, and porcine gelatine. The second soft capsule layer-forming solution is prepared by mixing cranberry extract, sodium alginate, and porcine gelatine.

A first soft capsule filing solution and a second soft capsule filling solution are prepared for the first soft capsule content and the second soft capsule content respectively. The first soft capsule filing solution is prepared by mixing a cranberry extract, prebiotic fibre, mint flavour, and tea tree oil. Tea tree oil is used as a solvent and has advantageous anti-microbial effects. The second soft capsule filing solution is prepared by mixing the cranberry extract and tea tree oil.

Subsequently, the second soft capsule filling solution is supplied into a first nozzle, the second soft capsule layer-forming solution is supplied into a second nozzle, the first soft capsule filling solution is supplied into a third nozzle, and then the first soft capsule layer-forming solution is supplied into an outermost fourth nozzle. The first, second, third and fourth nozzles are arranged concentrically and the diameter of the nozzles gradually increases in a numerical order. The multi-layered soft capsule according to an embodiment of the present invention is produced by simultaneously extruding the solutions through the annular ends of the nozzles, respectively, to make a four-phase composite jet stream, followed by releasing the jet stream into a cooling solution. The resulting soft capsule is then dried.

The soft capsules according to an embodiment of the present invention are spherical, light yellow colored gelatine capsules.

The weight of the second soft capsule layer is 8 mg. The weight of the second soft capsule content is 26 mg. The weight of the first soft capsule layer is 18 mg. The weight of the first soft capsule content is 209 mg.

The weight of the second soft capsule according to an embodiment of the present invention is 34 mg ± 4 mg. The weight of the soft capsule according to an embodiment of the present invention is 261 mg ± 26 mg. The weight is the average weight as determined by weighing 20 capsules with an electric balance and dividing the result by 20. Averaging is performed after repeating 3 times.

The diameter of the second soft capsule according to an embodiment of the present invention is 4.0 mm ± 0.2 mm. The diameter of the (first) soft capsule according to an embodiment of the present invention is 8.0 mm ± 0.2 mm. The diameter is the average diameter as determined by measuring 20 capsules in line with a ruler and dividing the result by 20. Averaging is performed after repeating 3 times.

The total aerobic bacteria of the soft capsule according to an embodiment of the present invention are not more than 1000 CFU/g.

The soft capsules according to an embodiment of the present invention can be blistered. The administration of a soft capsule according to an embodiment of the present invention can be one soft capsule per day to prevent and/or treat halitosis. Alternatively, the administration of a soft capsule according to an embodiment of the present invention can be when the subject is in need of a treatment for instance after consuming certain foods (e.g., garlic or onion) or certain drinks (e.g., alcohol).

The soft capsules according to an embodiment of the present invention have a shelf life of 6 months from the date of manufacturing in case of storing at dry and cool place (e.g., below 25°C and 55% humidity). To ensure the viability and shelf life, it is recommended to store the soft capsules illustrating the present invention at refrigerated temperature in original sealed package.

## Claims

1. A soft capsule comprising (a) a first soft capsule configured for targeted delivery to the mouth and (b) a second soft capsule configured for targeted delivery to the stomach, wherein the first soft capsule comprises one or more active ingredients capable of blocking adhesion of harmful bacteria to the oral cavity, a metal salt, and a prebiotic fibre, wherein the one or more active ingredients capable of blocking adhesion of harmful bacteria to the oral cavity are selected from the group consisting of cranberry extract, wild blueberry extract, strawberry extract, tea tree oil, and chamomile oil, and wherein the metal salt is a zinc salt; the second soft capsule comprises one or more active ingredients capable of blocking adhesion of harmful bacteria to the stomach and an alginate, wherein the one or more active ingredients capable of blocking adhesion of harmful bacteria to the stomach are selected from the group consisting of cranberry extract, wild blueberry extract, strawberry extract, tea tree oil, chamomile oil, tea extract, neem stick extract, snowdrop extract, seaweed extract, hop bract extract, Galla chinensis extract, avocado, and legume storage proteins; and the second soft capsule is contained in the first soft capsule.

2. The soft capsule according to claim 1, wherein the active ingredient capable of blocking adhesion of harmful bacteria to the oral cavity is cranberry extract.

3. The soft capsule according to claim 1 or 2, wherein the one or more active ingredients capable of blocking adhesion of harmful bacteria to the stomach are selected from the group consisting of cranberry extract, wild blueberry extract, strawberry extract, tea tree oil, and chamomile oil; preferably wherein the one or more active ingredients capable of blocking adhesion of harmful bacteria to the oral cavity or to the stomach are cranberry extract and tea tree oil.

4. The soft capsule according to claim 1 or 2, wherein the active ingredient capable of blocking adhesion of harmful bacteria to the stomach is selected from the group consisting of cranberry extract, tea extract, neem stick extract, snowdrop extract, seaweed extract, hop bract extract, Galla chinensis extract, avocado, and legume storage proteins; preferably wherein the active ingredient capable of blocking adhesion of harmful bacteria to the stomach is cranberry extract.

5. The soft capsule according to any one of claims 1 to 4, wherein the metal salt is a zinc salt selected from the group consisting of zinc acetate, zinc gluconate, zinc picolinate, zinc amino acid chelate, zinc chloride, zinc citrate, and zinc bisglycinate chelate; preferably wherein the metal salt is zinc acetate.

6. The soft capsule according to any one of claims 1 to 5, wherein the prebiotic fibre is selected from the group consisting of inulin, oligofructose, resistant starch, fructooligosaccharides (FOS), galactooligosaccharides (GOS), transgalactooligosaccharides (TOS), xylooligosaccharides (XOS), polydextrose, wheat dextrin, acacia gum, psyllium, banana, whole grain wheat, and whole grain corn; preferably wherein the prebiotic fibre is inulin.

7. The soft capsule according to any one of claims 1 to 6, wherein the alginate is selected from the group consisting of sodium alginate, potassium alginate, and calcium alginate; preferably wherein the alginate is sodium alginate.

8. The soft capsule according to any one of claims 1 to 7, wherein the first soft capsule comprises cranberry extract, a zinc salt, and a prebiotic fibre, preferably wherein the first soft capsule comprises from 1 mg to 150 mg by weight of the cranberry extract, from 1 mg to 10 mg by weight of the zinc salt, and from 1mg to 10 mg by weight of the prebiotic fibre.

9. The soft capsule according to any one of claims 1 to 8, wherein the second soft capsule comprises cranberry extract and alginate, preferably wherein the second soft capsule comprises from 1 mg to 15 mg by weight of the cranberry extract and from 1 mg to 15 mg by weight of the alginate.

10. The soft capsule according to any one of claims 1 to 9, wherein the first and/or second soft capsule further comprise an edible vegetable oil.

11. The soft capsule according to any one of claims 1 to 10, wherein the soft capsule is configured for oral administration.

12. The soft capsule according to any one of claims 1 to 11, for use as a medicament.

13. The soft capsule according to any one of claims 1 to 12, for use in the treatment of halitosis.

14. The soft capsule for use according to claim 13, wherein the treatment of halitosis comprises an oral treatment of halitosis and a gastrointestinal treatment of halitosis.

15. The soft capsule for use according to claim 14, wherein the gastrointestinal treatment of halitosis comprises a treatment of halitosis in the stomach.

16. A method for preparing a soft capsule according to any one of claims 1 to 11, comprising simultaneously extruding a composite jet stream into a cooling solution, wherein the composite jet stream consists of a second soft capsule filling solution extruded through a first nozzle, a second soft capsule layer-forming solution though a second nozzle, a first soft capsule filling solution extruded through a third nozzle and a first soft capsule layer-forming solution extruded through a fourth nozzle, with the first, second, third and fourth nozzles being arranged concentrically, a diameter of the nozzles gradually increasing in a numerical order, and wherein the first soft capsule layer-forming solution comprises a metal salt, wherein the metal salt is a zinc salt; the first soft capsule filling solution comprises one or more active ingredients capable of blocking adhesion of harmful bacteria to the oral cavity and a prebiotic fibre, wherein the one or more active ingredients capable of blocking adhesion of harmful bacteria to the oral cavity are selected from the group consisting of cranberry extract, wild blueberry extract, strawberry extract, tea tree oil, and chamomile oil; the second soft capsule layer-forming solution comprises an alginate; and the second soft capsule filling solution comprises one or more active ingredients capable of blocking adhesion of harmful bacteria to the stomach, wherein the one or more active ingredients capable of blocking adhesion of harmful bacteria to the stomach are selected from the group consisting of cranberry extract, wild blueberry extract, strawberry extract, tea tree oil, chamomile oil, tea extract, neem stick extract, snowdrop extract, seaweed extract, hop bract extract, Galla chinensis extract, avocado, and legume storage proteins.

## Patentansprüche

1. Weichkapsel, umfassend (a) eine erste Weichkapsel, die für die gezielte Verabreichung an den Mund konfiguriert ist, und (b) eine zweite Weichkapsel, die für die gezielte Verabreichung an den Magen konfiguriert ist, wobei die erste Weichkapsel ein oder mehrere Wirkstoffe, die fähig sind, die Adhäsion von schädlichen Bakterien an die Mundhöhle zu blockieren, ein Metallsalz und eine präbiotische Faser umfasst, wobei der eine bzw. die mehreren Wirkstoffe, die dazu fähig sind, die Adhäsion von schädlichen Bakterien an der Mundhöhle zu blockieren, aus der aus Cranberry-Extrakt, Wilde-Heidelbeere-Extrakt, Erdbeerextrakt, Teebaumöl und Kamillenöl bestehenden Gruppe ausgewählt sind und wobei es sich bei dem Metallsalz um ein Zinksalz handelt, die zweite Weichkapsel ein oder mehrere Wirkstoffe, die fähig sind, die Adhäsion von schädlichen Bakterien an den Magen zu blockieren, und ein Alginat umfasst, wobei der eine oder die mehreren Wirkstoffe, die dazu fähig sind, die Adhäsion von schädlichen Bakterien an den Magen zu blockieren, aus der aus Cranberry-Extrakt, Wilde-Heidelbeere-Extrakt, Erdbeerextrakt, Teebaumöl, Kamillenöl, Teeextrakt, Neemzweigextrakt, Schneeglöckchenextrakt, Algenextrakt, Hopfendeckblattextrakt, Galla-chinensis-Extrakt, Avocado und Leguminosenspeicherproteinen bestehenden Gruppe ausgewählt sind und die zweite Weichkapsel in der ersten Weichkapsel enthalten ist.

2. Weichkapsel nach Anspruch 1, wobei es sich bei dem Wirkstoff, der dazu fähig ist, die Adhäsion von schädlichen Bakterien an die Mundhöhle zu blockieren, um Cranberry-Extrakt handelt.

3. Weichkapsel nach Anspruch 1 oder 2, wobei der eine oder die mehreren Wirkstoffe, die dazu fähig sind, die Adhäsion von schädlichen Bakterien an den Magen zu blockieren, aus der aus Cranberry-Extrakt, Wilde-Heidelbeere-Extrakt, Erdbeerextrakt, Teebaumöl und Kamillenöl bestehenden Gruppe ausgewählt sind und wobei es sich bei dem einen oder den mehreren Wirkstoffen, die dazu fähig sind, die Adhäsion von schädlichen Bakterien an die Mundhöhle oder an den Magen zu blockieren, vorzugsweise um Cranberry-Extrakt und Teebaumöl handelt.

4. Weichkapsel nach Anspruch 1 oder 2, wobei der Wirkstoff, der dazu fähig ist, die Adhäsion von schädlichen Bakterien an den Magen zu blockieren, aus der aus Cranberry-Extrakt, Teeextrakt, Neemzweigextrakt, Schneeglöckchenextrakt, Algenextrakt, Hopfendeckblattextrakt, Galla-chinensis-Extrakt, Avocado und Leguminosenspeicherproteinen bestehenden Gruppe ausgewählt ist, wobei es sich bei dem Wirkstoff, der dazu fähig ist, die Adhäsion von schädlichen Bakterien an den Magen zu blockieren, vorzugsweise um Cranberry-Extrakt handelt.

5. Weichkapsel nach einem der Ansprüche 1 bis 4, wobei es sich bei dem Metallsalz um ein aus der aus Zinkacetat, Zinkgluconat, Zinkpicolat, Zinkaminosäurechelat, Zinkchlorid, Zinkcitrat und Zinkbisglycinatchelat bestehenden Gruppe ausgewähltes Zinksalz handelt, wobei es sich bei dem Metallsalz vorzugsweise um Zinkacetat handelt.

6. Weichkapsel nach einem der Ansprüche 1 bis 5, wobei die präbiotische Faser aus der aus Inulin, Oligofructose, resistenter Stärke, Fructooligosacchariden (FOS), Galactooligosacchariden (GOS), Transgalactooligosacchariden (TOS), Xylooligosacchariden (XOS), Polydextrose, Weizendextrin, Gummi arabicum, Psyllium, Banane, Vollkornweizen und Vollkornmais bestehenden Gruppe ausgewählt ist, wobei es sich bei der präbiotischen Faser vorzugsweise um Inulin handelt.

7. Weichkapsel nach einem der Ansprüche 1 bis 6, wobei das Alginat aus der aus Natriumalginat, Kaliumalginat und Calciumalginat bestehenden Gruppe ausgewählt ist, wobei es sich bei dem Alginat vorzugsweise um Natriumalginat handelt.

8. Weichkapsel nach einem der Ansprüche 1 bis 7, wobei die erste Weichkapsel Cranberry-Extrakt, ein Zinksalz und eine präbiotische Faser umfasst, wobei die erste Weichkapsel vorzugsweise 1 mg bis 150 mg nach Gewicht des Cranberry-Extrakts, 1 mg bis 10 mg nach Gewicht des Zinksalzes und 1 mg bis 10 mg nach Gewicht der präbiotischen Faser umfasst.

9. Weichkapsel nach einem der Ansprüche 1 bis 8, wobei die zweite Weichkapsel Cranberry-Extrakt und Alginat umfasst, wobei die zweite Weichkapsel vorzugsweise 1 mg bis 15 mg nach Gewicht des Cranberry-Extrakts und 1 mg bis 15 mg nach Gewicht des Alginats umfasst.

10. Weichkapsel nach einem der Ansprüche 1 bis 9, wobei die erste und/oder zweite Weichkapsel weiterhin ein essbares Pflanzenöl umfasst.

11. Weichkapsel nach einem der Ansprüche 1 bis 10, wobei die Weichkapsel für die orale Verabreichung konfiguriert ist.

12. Weichkapsel nach einem der Ansprüche 1 bis 11 zur Verwendung als Medikament.

13. Weichkapsel nach einem der Ansprüche 1 bis 12 zur Verwendung bei der Behandlung von Halitose.

14. Weichkapsel zur Verwendung nach Anspruch 13, wobei die Behandlung von Halitose eine orale Behandlung von Halitose und eine gastrointestinale Behandlung von Halitose umfasst.

15. Weichkapsel zur Verwendung nach Anspruch 14, wobei die gastrointestinale Behandlung von Halitose eine Behandlung von Halitose im Magen umfasst.

16. Verfahren zur Herstellung einer Weichkapsel nach einem der Ansprüche 1 bis 11, bei dem man gleichzeitig einen gemischten Jetstrahl in eine Kühllösung extrudiert, wobei der gemischte Jetstrahl aus einer Fülllösung für eine zweite Weichkapsel, die durch eine erste Düse extrudiert wird, einer eine Schicht für eine zweite Weichkapsel bildenden Lösung, durch eine zweite Düse, einer Fülllösung für eine erste Weichkapsel, die durch eine dritte Düse extrudiert wird, und einer eine Schicht für eine erste Weichkapsel bildenden Lösung, die durch eine vierte Düse extrudiert wird, besteht, wobei die erste, zweite, dritte und vierte Düse konzentrisch angeordnet sind, der Durchmesser der Düsen in numerischer Ordnung schrittweise zunimmt und wobei die eine Schicht für eine erste Weichkapsel bildende Lösung ein Metallsalz umfasst, wobei es sich bei dem Metallsalz um ein Zinksalz handelt, die Fülllösung für die erste Weichkapsel einen oder mehrere Wirkstoffe, die dazu fähig sind, die Adhäsion von schädlichen Bakterien an die Mundhöhle zu blockieren, und eine präbiotische Faser umfasst, wobei der eine oder die mehreren Wirkstoffe, die dazu fähig sind, die Adhäsion von schädlichen Bakterien an die Mundhöhle zu blockieren, aus der aus Cranberry-Extrakt, Wilde-Heidelbeere-Extrakt, Erdbeerextrakt, Teebaumöl und Kamillenöl bestehenden Gruppe ausgewählt sind, die eine Schicht für eine zweite Weichkapsel bildende Lösung ein Alginat umfasst, und die Fülllösung für die zweite Weichkapsel einen oder mehrere Wirkstoffe, die dazu fähig sind, die Adhäsion von schädlichen Bakterien an den Magen zu blockieren, umfasst, wobei der eine oder die mehreren Wirkstoffe, die dazu fähig sind, die Adhäsion von schädlichen Bakterien an den Magen zu blockieren, aus der aus Cranberry-Extrakt, Wilde-Heidelbeere-Extrakt, Erdbeerextrakt, Teebaumöl, Kamillenöl, Teeextrakt, Neemzweigextrakt, Schneeglöckchenextrakt, Algenextrakt, Hopfendeckblattextrakt, Galla-chinensis-Extrakt, Avocado und Leguminosenspeicherprotein bestehenden Gruppe ausgewählt sind.

## Revendications

1. Capsule souple comprenant (a) une première capsule souple conçue pour une administration ciblée à la bouche et (b) une deuxième capsule souple conçue pour une administration ciblée à l'estomac, la première capsule souple comprenant un ou plusieurs ingrédients actifs capables de bloquer l'adhérence de bactéries nuisibles à la cavité orale, un sel métallique, et une fibre prébiotique, l'ingrédient actif ou les ingrédients actifs capables de bloquer l'adhérence de bactéries nuisibles à la cavité orale étant choisis dans le groupe constitué par un extrait d'airelle, un extrait de myrtille sauvage, un extrait de fraise, une huile d'arbre à thé, et une huile de camomille, et le sel métallique étant un sel de zinc ; la deuxième capsule souple comprenant un ou plusieurs ingrédients actifs capables de bloquer l'adhérence de bactéries nuisibles à l'estomac et un alginate, l'ingrédient actif ou les ingrédients actifs capables de bloquer l'adhérence de bactéries nuisibles à l'estomac étant choisis dans le groupe constitué par un extrait d'airelle, un extrait de myrtille sauvage, un extrait de fraise, une huile d'arbre à thé, une huile de camomille, un extrait de thé, un extrait de tige de neem, un extrait de perce-neige, un extrait d'algues, un extrait de bractée de houblon, un extrait de Galla chinensis, l'avocat, et des protéines de stockage des légumineuses ; et la deuxième capsule souple étant contenue dans la première capsule souple.

2. Capsule souple selon la revendication 1, l'ingrédient actif capable de bloquer l'adhérence de bactéries nuisibles à la cavité orale étant un extrait d'airelle.

3. Capsule souple selon la revendication 1 ou 2, l'ingrédient actif ou les ingrédients actifs capables de bloquer l'adhérence de bactéries nuisibles à l'estomac étant choisis dans le groupe constitué par un extrait d'airelle, un extrait de myrtille sauvage, un extrait de fraise, une huile d'arbre à thé, et une huile de camomille ; préférablement l'ingrédient actif ou les ingrédients actifs capables de bloquer l'adhérence de bactéries nuisibles à la cavité orale ou à l'estomac étant un extrait d'airelle et une huile d'arbre à thé.

4. Capsule souple selon la revendication 1 ou 2, l'ingrédient actif capable de bloquer l'adhérence de bactéries nuisibles à l'estomac étant choisi dans le groupe constitué par un extrait d'airelle, un extrait de thé, un extrait de tige de neem, un extrait de perce-neige, un extrait d'algues, un extrait de bractée de houblon, un extrait de Galla chinensis, l'avocat, et des protéines de stockage des légumineuses ; préférablement l'ingrédient actif capable de bloquer l'adhérence de bactéries nuisibles à l'estomac étant un extrait d'airelle.

5. Capsule souple selon l'une quelconque des revendications 1 à 4, le sel métallique étant un sel de zinc choisi dans le groupe constitué par l'acétate de zinc, du gluconate de zinc, du picolinate de zinc, un chélate d'acide aminé de zinc, du chlorure de zinc, du citrate de zinc, et du chélate de bisglycinate de zinc ; préférablement le sel métallique étant l'acétate de zinc.

6. Capsule souple selon l'une quelconque des revendications 1 à 5, la fibre prébiotique étant choisie dans le groupe constitué par l'inuline, l'oligofructose, l'amidon résistant, les fructooligosaccharides (FOS), les galactooligosaccharides (GOS), les transgalactooligosaccharides (TOS), les xylooligosaccharides (XOS), le polydextrose, la dextrine de blé, la gomme d'acacia, le psyllium, la banane, le blé complet, et le maïs complet ; préférablement la fibre prébiotique étant l'inuline.

7. Capsule souple selon l'une quelconque des revendications 1 à 6, l'alginate étant choisi dans le groupe constitué par l'alginate de sodium, l'alginate de potassium, et l'alginate de calcium ; préférablement l'alginate étant l'alginate de sodium.

8. Capsule souple selon l'une quelconque des revendications 1 à 7, la première capsule souple comprenant un extrait d'airelle, un sel de zinc, et une fibre prébiotique, préférablement la première capsule souple comprenant de 1 mg à 150 mg en poids de l'extrait d'airelle, de 1 mg à 10 mg en poids du sel de zinc et de 1 mg à 10 mg en poids de la fibre prébiotique.

9. Capsule souple selon l'une quelconque des revendications 1 à 8, la deuxième capsule souple comprenant un extrait d'airelle et un alginate, préférablement la deuxième capsule souple comprenant de 1 mg à 15 mg en poids de l'extrait d'airelle et de 1 mg à 15 mg en poids de l'alginate.

10. Capsule souple selon l'une quelconque des revendications 1 à 9, la première et/ou la deuxième capsule souple comprenant en outre une huile végétale comestible.

11. Capsule souple selon l'une quelconque des revendications 1 à 10, la capsule souple étant conçue pour une administration orale.

12. Capsule souple selon l'une quelconque des revendications 1 à 11, pour une utilisation en tant que médicament.

13. Capsule souple selon l'une quelconque des revendications 1 à 12, pour une utilisation dans le traitement de l'halitose.

14. Capsule souple pour une utilisation selon la revendication 13, le traitement de l'halitose comprenant un traitement oral de l'halitose et un traitement gastro-intestinal de l'halitose.

15. Capsule souple pour une utilisation selon la revendication 14, le traitement gastro-intestinal de l'halitose comprenant un traitement de l'halitose dans l'estomac.

16. Procédé pour la préparation d'une capsule souple selon l'une quelconque des revendications 1 à 11, comprenant l'extrusion simultanée d'un courant-jet de composite dans une solution de refroidissement, le courant-jet de composite étant constitué d'une deuxième solution de remplissage de capsule souple extrudée à travers une première buse, une deuxième solution de formation de couche de capsule souple à travers une deuxième buse, une première solution de remplissage de capsule souple extrudée à travers une troisième buse et une première solution de formation de couche de capsule souple extrudée à travers une quatrième buse, les première, deuxième, troisième et quatrième buses étant disposées de manière concentrique, un diamètre des buses augmentant graduellement dans un ordre numérique, et la première solution de formation de couche de capsule souple comprenant un sel métallique, le sel métallique étant un sel de zinc ; la première solution de remplissage de capsule souple comprenant un ou plusieurs ingrédients actifs capables de bloquer l'adhérence de bactéries nuisibles à la cavité orale et une fibre prébiotique, l'ingrédient actif ou les ingrédients actifs capables de bloquer l'adhérence de bactéries nuisibles à la cavité orale étant choisis dans le groupe constitué par un extrait d'airelle, un extrait de myrtille sauvage, un extrait de fraise, une huile d'arbre à thé, et une huile de camomille ; la deuxième solution de formation de couche de capsule souple comprenant un alginate ; et la deuxième solution de remplissage de capsule souple comprenant un ou plusieurs ingrédients actifs capables de bloquer l'adhérence de bactéries nuisibles à l'estomac, l'ingrédient actif ou les ingrédients actifs capables de bloquer l'adhérence de bactéries nuisibles à l'estomac étant choisis dans le groupe constitué par un extrait d'airelle, un extrait de myrtille sauvage, un extrait de fraise, une huile d'arbre à thé, une huile de camomille, un extrait de thé, un extrait de tige de neem, un extrait de perce-neige, un extrait d'algues, un extrait de bractée de houblon, un extrait de Galla chinensis, l'avocat, et des protéines de stockage de légumineuses.
